# EUROPÄISCHE PATENTSCHRIFT

(11) **EP 0 550 118 B1**
(45) Veröffentlichungstag und Bekanntmachung des Hinweises auf die Patenterteilung: **24.07.1996**
(21) Anmeldenummer: 92250363.6
(22) Anmeldetag: 22.12.1992
(51) Int. Cl.: A61F 2/46, A61F 2/36

(54) **Ausschlaginstrument**
Endoprosthesis and appliance for driving it in or out
Endoprothèse et appareil pour l'enforcer ou l'extraire

(30) Priorität: 30.12.1991 DE 9116145 U; 25.06.1992 DE 4220970
(43) Veröffentlichungstag der Anmeldung: 07.07.1993
(73) Patentinhaber: ARTOS Medizinische Produkte GmbH, D-12277 Berlin (DE)
(72) Erfinder: Kranz, Curt, Dr.-Ing., W-1000 Berlin 62 (DE); Kahl, Susanne, Dipl.-Ing., W-1000 Berlin 38 (DE)
(74) Vertreter: Christiansen, Henning, Dipl.-Ing.

(56) Entgegenhaltungen:
- EP-A- 0 408 109
- WO-A-91/06262
- FR-A- 2 615 097
- DATABASE WPIL, AN: 83-E3023K, DERWENT PUBLICATIONS LTD., London, GB & SU-A-929 088

## Beschreibung

Die Erfindung betrifft ein Ausschlaginstrument der im Oberbegriff des Anspruchs 1 angegebenen Art sowie Knochenraspeln bzw. Schaftprothesen, die Zusammen mit dem erfindungsgemäßen Ausschlaginstrument in bevorzugter Weise verwendbar sind. Eine Variante eines derartigen Ausschlagelements kann auch zum Eintreiben von Schaftprothesen dienen.

Beim Einsetzen und Entfernen einer Schaftendoprothese kommt es darauf an, den Prothesenschaft fest zu ergreifen, um durch eine sichere Handhabung eine präzise Führung zu ermöglichen. Fuhr die vorbereitende Anpassung der Form des Markraums für den später einzusetzenden Prothesenschaft sind entsprechende Raspeln gebräuchlich, welche dem Prothesenschaft angepaßt sind und auch mit Anschlußmitteln für ein Instrument versehen sind, die denjenigen der Prothese geometrisch nachgebildet sind.

Beim Einsetzen einer Schaftendoprothese muß also zunächst eine Knochenhöhlung durch das Ausraspeln des Innenraumes des Knochens mit einer Knochenraspel, die der Form der einzusetzenden Schaftendoprothese angepaßt ist, gebildet werden, um dann die Schaftendoprothese in die Knochenhöhlung einsetzen zu können. Es sind auch Knochenraspeln bekannt, die als sogenannte "Probeprothesen" verwendet werden, in dem ihre Form die der Schaftendoprothese entspricht und sie mit einem Hals, der entsprechend dem Hals der einzusetzenden Schaftendoprothese ausgerichtet und ausgebildet sind und auf den die verschiedenen Prothesenköpfe aufgesetzt werden, um den Prothesenkopf wählen zu können, der die geeigneten Abmessungen aufweist.

Bei bekannten Einschlägern wird die proximale Fläche des Knochenraspel- bzw. Prothesenhalses als Einschlagfläche benutzt- und eine den Knochenraspel- bzw. Prothesenhals durchquerende Bohrung dient zur Einführung eines weiteren Instruments, mit dem die Knochenraspel bzw. dann Prothese wieder herausgezogen werden kann.

Nachteilig hierbei sind, daß der Knochenraspel- bzw. Prothesenhals durch die Querbohrung geschwächt wird und beim Einschlagen beschädigt werden kann. Ferner ist sowohl beim Ein- als auch beim Ausschlagen der Kräfteverlauf ungünstig, da beim Einschlagen die Schlagkraftübertragung nicht in Richtung des Knochenraspel- bzw. Prothesenschafts erfolgt, so daß Bereiche der Knochenhöhlung zu stark belastet werden. Beim Ausschlagen ist hingegen die Knochenraspel bzw. Prothese aufgrund der Hebelwirkung der nicht mit der Knochenraspel- bzw. Prothesenschaftlängsachse zusammenfallenden Wirkungsrichtung der Auschlagkraft nur unter einer zusätzlichen, zu einer Verkantung der Prothese führenden beträchtlichen Momentwirkung aus der Knochenhöhlung zu entfernen.

Bei einem aus der EP-A-0 175 079 bekannten Ein- bzw. Ausschlaginstrument ist eine Gabel am Ende des Instrumentenschafts befestigt. Die Gabelzinken weisen Rippen und der Prothesenhals entsprechende Nuten auf, so daß beim Ansetzen des Instruments die Rippe in die Nut eingreift. Das Ende des Instrumentenschafts ist mit einem Gewinde versehen und wird von einer Gewindebohrung in die Gabel aufgenommen. Die herausragende Spitze des Schafts wird nach dem Ansetzen der Gabel am Hals durch das Anziehen des Gewindes in eine Arretierbohrung im Prothesenkragen eingesetzt.

Nachteilig bei diesem bekannten Ein- bzw. Ausschlaginstrument ist jedoch, daß aufgrund seiner geometrischen Gestaltung seine Handhabung in der Operationsphase - und insbesondere Ausrichtung - durch Körperweichteile behindert werden kann.

Aus FR-A-2 615 097 ist ein gattungsgemäßes Ausschlaginstrument bekannt, bei dem der Hals der Prothese durch ein in Längsrichtung des Instrumentenschaftes axial verschiebliches, über einen Drehgriff am oberen Ende des Instrumentes - über den zugleich das Aus- bzw. Einschlagen der Prothese erfolgt - betätigtes Klemmelement kraft- und formschlüssig lösbar mit dem Instrument verbunden werden kann.

Der Erfindung liegt demgegenüber die Aufgabe zugrunde, ein Ausschlaginstrument der eingangs genannten Gattung derart weiterzubilden, daß eine zuverlässige Funktion und hohe Lebensdauer erreicht werden. Diese Aufgabe wird mit den kennzeichnenden Merkmalen des Anspruchs 1 gelöst.

Durch die erfindungsgemäßen Maßnahmen wird eine Verbindungsgeometrie geschaffen, welche die Schaffung eines Instruments ermöglicht, welches wegen seiner schlanker Bauform durch benachbarte Weichteile bei der Benutzung nicht oder nur wenig behindert wird. Gleichzeitig sind auch die Momente verringert, so daß die beim Einschlagen zwangsläufig auftretenden, quer zur Einschlagrichtung verlaufenden Reaktionskräfte minimiert sind.

Das bewegliche Element, welches die Zunge trägt, ist bei einer bevorzugten Ausführungsform des Ein- bzw. Ausschlägers oberhalb der Halterung in einer parallel zur Raspel- bzw. Prothesenschaftachse gerichteten Ebene drehbar an der Halterung gelagert. Zum Festklemmen der Raspel bzw. Prothese dient ein Betätigungselement, welches bevorzugt am Instrumentenschaft als Rändelzylinder ausgebildet ist und mit dem beweglichen Element derart zusammenwirkt, daß bei Drehung des Rändelzylinders die Kante des letzteren so weit in Richtung der Halterung bewegt wird, bis das dadurch um die Drehachse geschwenkte bewegliche Element am Raspel- bzw. Prothesenhals anschlägt.

Entsprechend einer anderen günstigen Ausführungsform der Erfindung ist das Betätigungselement zum Festklemmen der Raspel bzw. Prothese als ein oberhalb des Drehpunktes an dem beweglichen Element befestigtes und bezüglich der Längsachse des Instrumentenschaftes quer verschiebliches Griffstück ausgebildet. Die Verschieblichkeit wird begrenzt durch das Anschlagen des beweglichen Elements am Prothesenhals. Die Verwendung eines solchen Griffstücks ist wegen seiner bequemen Handhabbarkeit und für das Erreichen eines hohen Anpreßdruckes des beweglichen Elements am Raspel- bzw. Prothesenhals von erheblichem Vorteil.

Das Betätigungselement verhindert gleichzeitig auch das unbeabsichtigte Zurücksetzen des beweglichen Elements nach Erreichen der gewünschten Position. Dies ist bei Verwendung eines an seinem freien Ende durch mechanische Mittel verriegelbaren Griffstücks besonders einfach zu realisieren.

Bei einer weiteren vorteilhaften Weiterbildung des beweglichen Elements weist dieses eine noppenartige Erhöhung auf, die einen zusätzlichen Anschlag bildet. Andernfalls wurde der Raspel- bzw. Prothesenhals sich beim Ein- oder Ausschlagen an die Seitenfläche der Halterung und an die noppenartige Erhöhung des beweglichen Elements anlegen, wobei diese derart am beweglichen Element angeordnet ist, daß sie etwa in halber Raspel- bzw. Prothesenhalshöhe liegt. Ein weiteres Herausgleiten wird somit verhindert, da der obere Teil des Raspel- bzw. Prothesenhalses nicht an der noppenartigen Erhebung vorbeigleiten kann.

Bei einer anderen vorteilhaften Weiterbildung des erfindungsgemäßen Ein- bzw. Ausschlägers weist dieser an seiner mit dem Raspel- bzw. Prothesenkragen zusammenwirkenden Fläche eine rillenförmige Erhöhung auf, die in eine entsprechende in der Fläche des Raspel- bzw. Prothesenkragens vorgesehene Nut eingreift. Dadurch wird eine reib-, form- und kraftschlüssige Verbindung zwischen Ein- bzw. Ausschläger und Raspel bzw. Prothese stets gewährleistet, und das geführte Einsetzen der Raspel bzw. Prothese in die ringförmige Öffnung der Halterung wird ermöglicht.

Bei einer weiteren bevorzugten Weiterbildung des erfindungsgemäßen Instruments ist die Flache des Elements, das am Raspel- bzw. Prothesenhals anliegt, entsprechend der konusförmigen Ausbildung des Raspel- bzw. Prothesenhalses als konkave, bogenförmige Flache, ausgebildet, so daß sie ganzflächig aufliegt und somit die Festigkeit der Klemmverbindung erhöht. Die mediale innere Seitenfläche der ringförmigen Öffnung kann ebenfalls entsprechend der Form des konusförmigen Raspel- bzw. Prothesenhalses konkav und bogenförmig ausgebildet sein, um die Klemmverbindung zu verbessern.

Mit dem erfindungsgemäßen Ein- bzw. Ausschläger für daran angepaßte Knochenraspeln und Prothesen kann mit einer Knochenraspel, die gleichzeitig als Probierprothese zur Reposition dient, eine zentrale präzise Fixierung des Prothesenschafts im Zementmantel nach Beendigung des Raspelvorgangs auf einfache Weise erzielt werden.

Da die Langzeitstabilität von zementierten Hüftendoprothesen von einer möglichst physiologischen Knochenbelastung und der Vermeidung von Überbelastungen des Zementmantels abhängt und die Voraussetzung dafür eine fehlstellenfreie und nach strukturmechanischen Gesichtspunkten erforderliche Dickenverteilung des Zementes sowie eine beständige Anbindung an Knochen und Prothese ist und man mit der zentralen präzisen Fixierung des Prothesenschafts eine optimierte Zementverteilung erreichen kann, ist die zu erwartende Langzeitstabilität einer mit einem erfindungsgemäßen Einschläger in eine vorgeraspelten Knochenhöhlung eingesetzten Prothese groß.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine bevorzugte Ausführungsform der erfindungsgemäßen Ein- bzw. Ausschlagvorrichtung und eine in den Ein- bzw. Ausschläger einzusetzende Prothese bzw. Raspel als Detail in Seitenansicht,
Figur 2 einen Längsschnitt durch die Ein- bzw. Ausschlagvorrichtung gemäß Figur 1 bei mit dieser verbundener Prothese bzw. Raspel,
Figur 3 eine weitere Ausführungsform der Erfindung als Seitenansicht mit dem Kopf einer entsprechend zu entfernenden Prothese,
Figur 4 die Seitenansicht von links der in Figur 3 dargestellten Vorrichtung
   sowie
Figur 5 eine Seitenansicht der in Figur 3 dargestellten Vorrichtung mit umfaßtem Prothesenkopf.

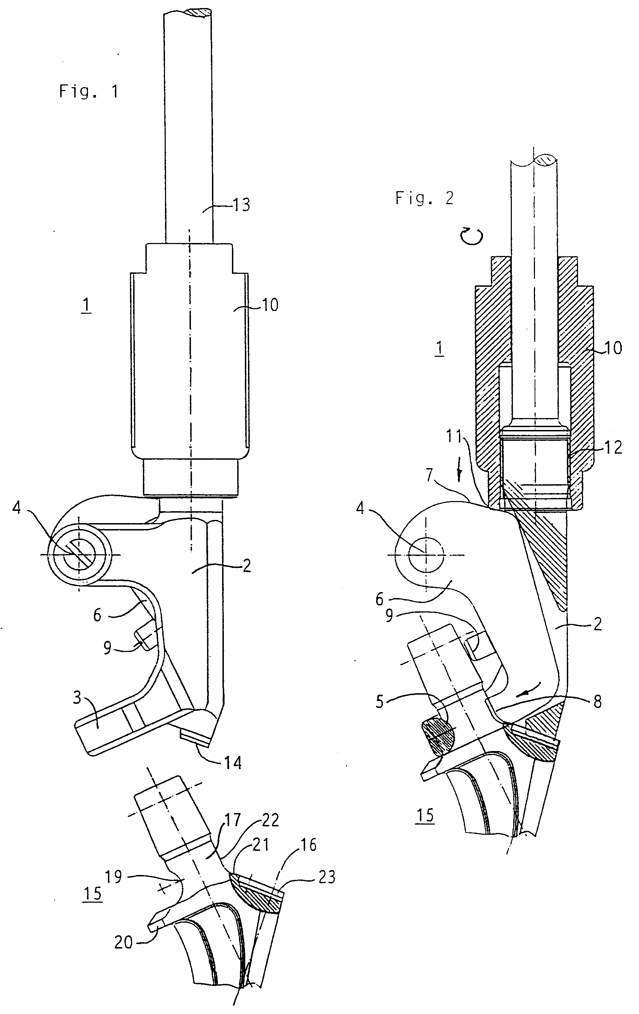

In den Figuren 1 und 2 ist jeweils in Seitenansicht eine bevorzugte Ausführung des erfindungsgemäßen Ein- bzw. Ausschlaginstrument 1 im Zusammenwirken mit einer Schaftprothese 15 dargestellt. Anstelle einer Prothese kann auch eine Raspel mit dem erfindungsgemäßen Instrument geführt werden, wenn sie entsprechend der Schaftprothese geformt ist und auch Anschlußmittel für den Ein- bzw. Ausschläger aufweist, welche entsprechend dem Halsbereich der Schaftprothese geformt sind. (Zur Erhöhung der Übersichtlichkeit der Zeichnung sind die freien Schaftenden von Prothese und Ein- bzw. Ausschlaginstruments geschnitten dargestellt).

Das in Figur 1 in seiner Position zum Ergreifen der Prothese bzw. Raspel wiedergegebene Ein- bzw. Ausschlaginstrument 1 weist einen Instrumentenschaft 13 auf, an dessen oberem Ende ein - nicht dargesteller - eine Schlagfläche aufweisender Handgriff und an dessen unterem Ende eine starr mit dem Griffende verbundene und diese in gerader Richtung fortsetzende Halterung 2 zur Aufnahme einer Prothese 15 bzw. einer entsprechenden Raspel vorgesehen ist.

Ein unterer, abgewinkelter, ringförmiger und starr mit der Halterung 2 verbundener Ansatz 3, welcher an die Halterung 2 in Fortsetzung der Richtung des Instrumentenschafts 13 anschließt, weist eine im wesentlichen runde Öffnung als Durchlaß für den Prothesen bzw. Raspelhals auf. Die Halterung 2 ist derart abgewinkelt, daß die Öffnung des abgewinkelten Ansatzes 3 den Hals einer Prothese umgreifen kann, deren Schaft sich in im wesentlichen geradliniger Fortsetzung des Instrumentenschafts 1 erstreckt. Die Richtung des ringförmigen Ansatzes bildet also mit der radialen Richtung des Instrumentenschaftes einen Winkel, der im wesentlichen der Abwinklung des Prothesenschaftes im Hauptkrümmungsbereich entspricht.

Der von der geometrischen Mittelachse des Instrumentenschafts 13 weg weisende Teil des ringförmigen Ansatzes 3 ist in seinem inneren Bereich 5 - bezogen auf den Ringquerschnitt - derart konvex verrundet ausgestaltet, daß er formschlüssig in eine entsprechend geformte konkave, an der Innenseite der Krümmung der Prothese gelegene Ausnehmung 19 des Prothesen- bzw. Raspelhalses 17 eingreift, nachdem der Prothesen- oder Raspelhals 17 in die Öffnung des abgewinkelten Ansatzes 3 eingeführt worden ist.

Innerhalb einer entsprechenden Ausnehmung der Halterung 2 ist ein um eine Achse 4 einseitig drehbar gelagerter Schwenkhebel 6 vorgesehen, der an seinem freien Ende eine in eine entsprechend geformte Ausnehmung 22 eingreifende Zunge 8 aufweist.

Der in die Halterung 2 eingeführte Prothesen- oder Raspelhals 17 ist somit mittels eines zwischen einer innerhalb der Halterung 2 um eine Achse 4 einseitig drehbar gelagerten Schwenkhebels 6 und der medialen inneren Seitenfläche 5 des Ansatzes 3 festklemmbar, wobei die Klemmposition des Schwenkhebels 6 mittels eines am Instrumentenschaft 1 angebrachten Betätigungselements 10 einstellbar ist.

Bei der dargestellten Ausführungsform des erfindungsgemäßen Ein- bzw. Ausschlägers ist das am Instrumentenschaft 1 angeordnete Betätigungselement 10 als Rändelzylinder ausgebildet und wirkt mit einem auf dem Instrumentenschaft angebrachten Gewind 12 derart zusammen, daß bei einer Drehung des Rändelzylinders 10 seine an einer Schulter 7 des Schwenkhebels 6 anliegende Kante die Zunge 8 des Hebels 6 in Richtung der entsprechend gestalteten weiteren Ausnehmung 22 des in den ringförmigen Ansatz 3 der Halterung 2 eingeführten Prothesen- bzw. Raspelhalses 15 bewegt und diese vor einem Zurücksetzen nach Erreichung der gewünschten Position hindert. Der Schwenkhebel bildet dabei eine Wegübersetzung für die Anpreßkraft der Kante 11, wobei der Weg der Zunge 8 im Vergleich zum Vorschubweg (Pfeil) in Bezug auf durch dessen Rotation (Pfeil) infolge des Gewindes 12 bewirkten Vorschubweg des Rändelzylinders 10 (Pfeil) heraufgesetzt wird.

Weiterhin weist der Schwenkhebel 6 in einem der Drehachse 4 näherliegenden Bereich eine einen zusätzlichen Anschlag bildende noppenartige Erhöhung 9 auf, die als weitere Sicherung gegen ein unbeabsichtigtes Herausgleiten des Prothesen- oder Raspelhalses 17 aus der Öffnung 3 der Halterung 2 bzw. als Anschlag zur Aufnahme von beim Ein- bzw. Ausschlagen des Prothesenschafts auftretenden Momenten dient. Eine konvexe Führungsrippe 14 dient im Zusammenwirken mit einer entsprechend ausgestalteten, im Bereich der Stirnfläche 21 des Kragens 20 der Schaftprothese 15 vorgesehenen, entsprechend geformten konkaven Ausnehmung (Auschlagfläche) 23 vorzugsweise zur Einleitung von Einschlagkräften in den Prothesenschaft.

In Figur 2 ist ein Längsschnitt durch die Halterung 2 des Ein- bzw. Ausschlägers der Figur 1 mit eingesetzter Prothese 15 dargestellt. Die Beschreibung bezieht sich entsprechend auch auf die Handhabung einer Raspel.

Es ist ersichtlich, daß die Ausnehmung 23 des Prothesen- oder Raspelkragens 20 und die untere Führungsrippe 14 der Halterung 2 formschlüssig ineinandergreifen, wenn die innere Anschlagfläche 5 des den Prothesenhals 18 umgreifenden abgewinkelten Ansatzes 3 in der Ausnehmung 19 des Halses anliegt und durch Schrauben des Rändelringes 10 in Richtung auf die Halterung 2 der Schwenkhebel 6 mit seiner Zunge 8 in die Ausnehmung 22 des Prothesenhalses 17 gepreßt wird. Die Frontfläche des zusätzlichen Anschlags 9 liegt dabei zusätzlich unterstützend im Bereich des die Gelenkkugel aufnehmenden Konusanschlusses 18 des Halses 17 an.

Auf diese Weise ist sichergestellt, daß beim Einschlagen trotz des gekrümmten Prothesenschaftes die Einschlagkräfte im wesentlichen geradlinig über die Anschlagfläche 23 in den Prothesenschaft eingeleitet werden. Ein seitliches Ausweichen von Werkzeug und Schaft ist einerseits durch die radial konvex geformte Rippe 14 im Zusammenwirken mit der entsprechend sattelförmig konkav geformten Ausnehmung 23, zum anderen aber durch das Umgreifen des Prothesenhalses durch den ringförmigen abgewinkelten Ansatz 3 unter Arretierung durch den Schwenkwinkel 6 gesichert.

Durch das Zusammenwirken der Anschläge 14, 8 und 9 werden auch die bei den auftretenden Ein- bzw. Ausschlagskräften auftretenden Reaktionsmomente unter allen Bedingungen sicher aufgenommen.

Die Ein- und Ausschlagimpulse selbst werden durch eine (in der Zeichnung nicht dargestellte) auf dem Instrumentenschaft 13 verschiebliche Masse aufgebracht, die entweder auf die obere Stirnfläche des Gewichts 10 oder aber auf eine entsprechende Anschlagfläche am oberen Ende des Schafts (in der Zeichnung ebenfalls nicht sichtbar) wirkt.

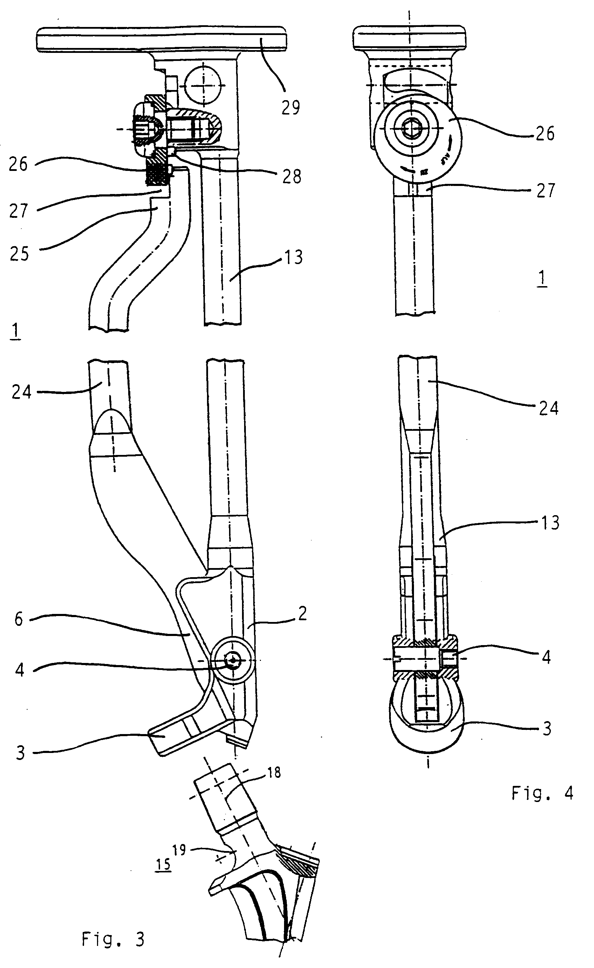

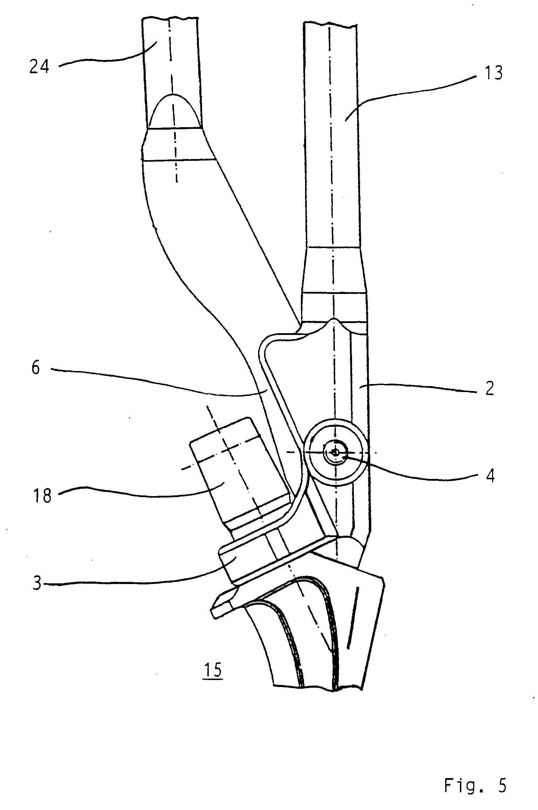

In den Figuren 3, 4 und 5 ist jeweils in Seitenansicht eine weitere bevorzugte Ausführung des erfindungsgemäßen Ein- bzw. Ausschlaginstrument 1 im Zusammenwirken mit einer Schaftprothese 15 dargestellt. Anstelle einer Prothese kann auch hier eine Raspel mit dem erfindungsgemäßen Instrument geführt werden, wenn sie entsprechend der Schaftprothese geformt ist und auch Anschlußmittel für den Ein- bzw. Ausschläger aufweist, welche entsprechend dem Halsbereich der Schaftprothese gestaltet sind.

Der innerhalb einer entsprechenden Ausnehmung der Halterung 2 um eine Achse 4 einseitig drehbar gelagerte Schwenkhebel 6 weist als Betätigungselement an seinem oberhalb der Drehachse 4 befindlichen Ende ein Griffstück 24 auf. Am freien Ende 25 des Griffstücks 24 ist eine Verriegelungsvorrichtung vorgesehen, die im wesentlichen aus einem Drehglied 26 besteht, das in seiner Verriegelungsposition das freie Ende 25 des Griffstücks 24 hintergreift. Das Griffstück 24 wird im wesentlichen parallel zur Achse des Instrumentenschaftes 13 bis zu dessen Ende geführt. An dem in Richtung des Schaftes 13 abgewinkelten freien Ende 25 des Griffstücks 24 ist eine Ausnehmung 27 vorgesehen. In diese kann das, am Instrumentenschaft 13 befestigte Drehglied 26 eingreifen, wenn das Griffstück 24 in Richtung des Schaftes 13 bewegt worden ist. Der in die Halterung 2 eingeführte Prothesen- oder Raspelhals 18 ist somit mittels eines innerhalb der Halterung 2 um eine Achse 4 einseitig drehbar gelagerten Schwenkhebels 6 und der medialen inneren Seitenfläche des Ansatzes 3 festklemmbar, wobei die Klemmposition des Schwenkhebels 6 mittels des Griffstücks 24 im Zusammenwirken mit dem Drehglied 26 eingestellt werden kann. Das Drehglied 26 ist am Schaft 13 befestigt und als exzentrisch gelagerte Scheibe ausgebildet. Die Größe der Stirnfläche des Drehglieds 26 nimmt dabei drehwinkelabhängig in Richtung auf die Verdrehungsposition tangential zu. Dadurch wird der Schwenkhebel 6 bei Fixieren des Griffstücks 24 durch das Drehglied 26, dessen Schwenkbreich durch Anschlagnasen 28 begrenzt ist, besonders fest an den Prothesenhals 18 gepreßt. Die Verwendung eines derartig verspannbaren Griffstücks 24 ist für eine einfache Handhabung des Ausschlaginstruments 1 besonders vorteilhaft und verhindert in günstiger Weise ein zwischenzeitliches Lösen der Klemmverbindung zwischen Prothesen- bzw. Raspelhals 18 und Halterung 2.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Ausschlaginstrument (1) für eine Gelenkprothese (15) mit einem zur Aufnahme der Gelenkkugel dienenden Prothesenhals (17) und mindestens einer Anschlagfläche (19), mit
einer am Schaft (13) des Instruments angebrachten, mit einem Durchlaß (3) für den Prothesenhals (17) versehenen und diesen im wesentlichen umgreifenden Halterung (2),
einer Anschlagfläche (5) an der Innenseite der Halterung (2) und
einer der Anschlagfläche (5) gegenüberliegenden beweglichen Zunge (8), welche zwecks kraftschlüssiger Arretierung des Instruments bezüglich der Gelenkprothese (15) mittels am Instrument vorgesehener Betätigungsmittel (10, 24) in einer Richtung verschieblich ist, welche eine in bezug auf den Prothesenhals (17) radiale Richtungskomponente aufweist,
**dadurch gekennzeichnet,** daß die Zunge (8) an einem schwenkbaren Element (6) befestigt ist, welches als ein um eine oberhalb der Anschlagfläche (5) der Halterung (2) gelegene Drehachse (4) verdrehbarer Schwenkhebel ausgebildet ist.

2. Ausschlaginstrument nach Anspruch 1, **dadurch gekennzeichnet,** daß die Form der Zunge (8) und die Form der Anschlagfläche (5) mit der Form der jeweils mit ihnen in Wechselwirkung tretende Fläche (22) des Prothesenhalses (17) in gegenseitiger Formanpassung aufeinander abgestimmt sind.

3. Ausschlaginstrument nach einem der vorangehenden Ansprüche, **dadurch gekennzeichnet,** daß die Zunge (8) in einer Ebene verschieblich gelagert ist, die der Hauptkrümmungsebene der Prothese (15) entspricht und daß sie insbesondere - bezogen auf die Hauptkrümmung der Prothese - von außen her auf diese einwirkt.

4. Ausschlaginstrument nach einem der vorangehenden Ansprüche, **dadurch gekennzeichnet,** daß die Zunge - bezogen auf die geometrische Mittelachse des Schafts (13) des Instruments - mit einer radial nach außen gerichteten Kraftkomponente auf den Prothesenhals (17) wirkt.

5. Ausschlaginstrument nach einem der vorangehenden Ansprüche 5, **dadurch gekennzeichnet,** daß das, die Zunge (8) tragende schwenkbare Element (6) als Betätigungsmittel ein Griffstück (24) aufweist, das oberhalb des Drehpunktes (4) an dem Element (6) befestigt ist und das sich im wesentlichen parallel zur geometrischen Längsachse des Schafts (13) des Instruments (1) erstreckt, wenn das Ausschlaginstrument (1) am Prothesenhals (17) angebracht worden ist.

6. Ausschlaginstrument nach Anspruch 5, **dadurch gekennzeichnet,** daß am freien Ende (25) des Griffstücks (24) eine Verriegelungsvorrichtung vorgesehen ist.

7. Ausschlaginstrument nach Anspruch 6, **dadurch gekennzeichnet,** daß die Verriegelungsvorrichtung ein exzentrisch gelagertes Drehglied (26) aufweist, welches in seiner Verriegelungsposition das freie Ende (25) des Griffstücks (24) hintergreift, wobei zur Drehwinkelbegrenzung mindestens eine Nocke (28) vorgesehen ist.

8. Ausschlaginstrument nach Anspruch 7, **dadurch gekennzeichnet,** daß das Drehglied (26) am Instrumentenschaft (13) befestigt ist und das freie Ende (25) des Griffstücks (24) eine Aussparung (27) aufweist.

9. Ausschlaginstrument nach einem der Ansprüche 7 oder 8, **dadurch gekennzeichnet,** daß das Drehglied (26) im wesentlichen scheibenförmig ausgebildet ist und die Größe seiner Stirnfläche drehwinkelabhängig in Richtung auf die Verriegelungsposition tangential zunimmt.

10. Ausschlaginstrument nach einem der vorangehenden Ansprüche, **dadurch gekennzeichnet,** daß das die Zunge (8) tragende schwenkbare Element (6) einen zusätzlichen oberhalb der Zunge (8) gelegenen, mit dem Prothesen- bzw. Knochenraspelhals (17) in Wechselwirkung tretenden Anschlag (9) aufweist, der insbesondere in Form einer Schulter ausgestaltet ist.

11. Ausschlaginstrument nach einem der Ansprüche 5 oder 11, **dadurch gekennzeichnet,** daß das schwenkbare Element (6) um eine Achse drehbar gelagert ist, die senkrecht zur geometrischen Mittelachse des Schafts (13) des Instruments gerichtet - und insbesondere radial dazu versetzt - ist.

12. Ausschlaginstrument nach einem der vorangehenden Ansprüche, **dadurch gekennzeichnet,** daß das schwenkbare Element (6) eine im Bereich des Instrumentenschaftes (13) gelegene Anschlagfläche (7) aufweist, auf der sich die Kante (11) eines in Richtung des Schaftes (13) beweglichen Betätigungselements (10) abstützt, wobei die Kante (11) bei Bewegung des Betätigungselements (10) in Richtung zur Halterung (2) hin bewegt wird und das schwenkbare Element (6) derart antreibt, daß die Zunge (8) gegen den Prothesenhals (17) gepreßt wird.

13. Ausschlaginstrument nach Anspruch 12, **dadurch gekennzeichnet,** daß das am Schaft (13) angeordnete Betätigungselement als mit einem Innengewinde (12) versehener Rändelzylinder (10) ausgebildet ist, dessen Kante (11) mit der Anschlagfläche (7) des die Zunge (8) tragenden schwenkbaren Elements (6) derart in Wechselwirkung steht, daß sie bei Bewegung des Ränderzylinders (10) in Richtung Halterung (2) bewegt wird und das schwenkbare Element (6) derart antreibt, daß die Zunge (8) gegen den Prothesenhals (17) gepreßt wird.

14. Ausschlaginstrument nach einem der vorangehenden Ansprüche, **dadurch gekennzeichnet,** daß eine zusätzliche Anschlagfläche (14) vorgesehen ist, mit der das Ausschlaginstrument auch als Einschlaginstrument verwendbar ist.

15. Ausschlaginstrument nach Anspruch 14, **dadurch gekennzeichnet,** daß die zusätzliche Anschlagfläche (14) in Verlängerung der geometrischen Mittelachsen von Prothesen- und Instrumentenschaft gelegen ist.

16. Ausschlaginstrument nach einem der Ansprüche 14 oder 15, **dadurch gekennzeichnet,** daß die zusätzliche Anschlagfläche (14) rippenförmig ausgebildet ist.

17. Ausschlaginstrument nach einem der Ansprüche 14 bis 16, **dadurch gekennzeichnet,** daß die zusätzliche Anschlagfläche (14) in eine Anschlagfläche (23) der Prothese (15) eingreift, welche auf der Außenseite der meximalen Krümmung des Schafts im Bereich der Stirnfläche des Prothesenschafts gelegen ist.

18. Ausschlaginstrument nach einem der vorangehenden Ansprüche, **gekennzeichnet durch** die Anwendung auf eine mit den einer Prothese entsprechenden Anschlußmitteln versehenen Knochenraspel.

## Claims

1. Instrument (1) for driving out a joint prosthesis (15) with a neck (17) serving to accommodate the ball of the joint, and at least one abutment surface (19), having a holder (2) mounted on the shaft (13) of the instrument, said holder being provided with an opening (3) for the neck (17) of the prosthesis and substantially embracing said neck (17), having an abutment surface (5) on the inside of the holder (2) and having a movable tongue (8) opposite the abutment surface (5), said tongue being movable, for frictional locking of the instrument relative to the joint prosthesis (15), by means of actuating means (10, 24) provided on the instrument, in a direction which has a radial component relative to the neck (17) of the prosthesis,
characterised in that the tongue (8) is attached to a pivotable element (6) which is constructed as a rocking lever rotatable about a rotation axis (4) located above the abutment surface (5) of the holder (2).

2. Instrument according to claim 1, characterised in that the shape of the tongue (8) and the shape of the abutment surface (5) are respectively adapted to the shape of the surface (22) of the neck (17) of the prosthesis which interacts with them.

3. Instrument according to one of the preceding claims, characterised in that the tongue (8) is movably mounted in a plane which corresponds to the main plane of curvature of the prosthesis (15) and in that, in particular, it acts thereon from the outside relative to the main curvature of the prosthesis.

4. Instrument according to one of the preceding claims, characterised in that the tongue acts on the neck (17) of the prosthesis with a radially outwardly directed force component, relative to the geometric central axis of the shaft (13) of the instrument.

5. Instrument according to one of the preceding claims, characterised in that the pivotable element (6) which carries the tongue (8) has as actuating means a handle portion (24) which is fixed to the element (6) above the pivot (4) and which extends substantially parallel to the geometric longitudinal axis of the shaft (13) of the instrument (1) when the instrument (1) has been fitted to the neck (17) of the prosthesis.

6. Instrument according to claim 5, characterised in that a locking device is provided at the free end (25) of the handle portion (24).

7. Instrument according to claim 6, characterised in that the locking device has an eccentrically mounted rotary member (26) which, in its locking position, engages behind the free end (25) of the handle portion (24), at least one cam (28) being provided in order to limit the angle of rotation.

8. Instrument according to claim 7, characterised in that the rotary member (26) is fixed to the instrument shaft (13) and the free end (25) of the handle portion (24) has a recess (27).

9. Instrument according to one of claims 7 or 9, characterised in that the rotary member (26) is substantially disc-shaped and the size of its outer face increases tangentially, as a function of the angle of rotation, towards the locking position.

10. Instrument according to one of the preceding claims, characterised in that the pivotable element (6) which carries the tongue (8) has an additional protuberance (9), particularly in the form of a shoulder, located above the tongue (8) and interacting with the neck (17) of the prosthesis or bone file.

11. Instrument according to one of claims 5 and 11, characterised in that the pivotable element (6) is mounted to be rotatable about an axis which is directed perpendicularly to the geometric central axis of the shaft (13) of the instrument and in particular radially offset relative thereto.

12. Instrument according to one of the preceding claims, characterised in that the pivotable element (6) has an abutment surface (7) located in the region of the shaft (13) of the instrument, on which the edge (11) of an actuating member (10) movable in the direction of the shaft (13) rests, the edge (11) being moved towards the holder (2) as the actuating element (10) moves, thereby driving the pivotable element (6) in such a way that the tongue (8) is pressed against the neck (17) of the prosthesis.

13. Instrument according to claim 12, characterised in that the actuating member mounted on the shaft (13) is constructed as a knurled cylinder (10) having an internal thread (12), the edge (11) of which interacts with the abutment surface (7) of the pivotable element (6) carrying the tongue (8) in such a way that, as the knurled cylinder (10) moves, the edge (11) is moved towards the holder (2) and drives the pivotable element (6) in such a way that the tongue (8) is pressed against the neck (17) of the prosthesis.

14. Instrument according to one of the preceding claims, characterised in that an additional abutment surface (14) is provided by means of which the instrument can also be used as an insertion instrument.

15. Instrument according to claim 14, characterised in that the additional abutment surface (14) is located on an extension of the geometric central axes of the shafts of the prosthesis and instrument.

16. Instrument according to one of claims 14 or 15, characterised in that the additional abutment surface (14) is of rib-like construction.

17. Instrument according to one of claims 14 to 16, characterised in that the additional abutment surface (14) engages in an abutment surface (23) of the prosthesis (15) which is located on the outside of the maximum curvature of the shaft in the region of the end surface of the prosthesis shaft.

18. Instrument according to one of the preceding claims, characterised by its use on a bone file provided with connection means corresponding to a prosthesis.

## Revendications

1. Instrument d'extraction (1) pour une prothèse d'articulation (15), comportant un col de prothèse (17) servant à recevoir la sphère d'articulation, et au moins une surface de butée (19), présentant :
- une monture (2) agencée sur la tige (13) de l'instrument, pourvue d'un passage (3) pour le col de prothèse (17) et entourant sensiblement celui-ci,
- une surface de butée (5) sur la face intérieure de la monture (2), et
- une languette (8) située en vis-à-vis de la surface de butée (5), qui est mobile dans une direction par l'intermédiaire de moyens d'actionnement (10, 24) prévus sur l'instrument, pour l'arrêt en coopération de forces de l'instrument par rapport à la prothèse d'articulation (15), qui présente une composante directionnelle radiale par rapport au col de prothèse (17), caractérisé en ce que la languette (8) est fixée sur un élément basculant (6) qui est réalisé sous la forme d'un levier basculant en rotation autour d'un axe de rotation (4) situé au-dessus de la surface de butée (5) de la monture (2).

2. Instrument d'extraction selon la revendication 1, caractérisé en ce que la forme de la languette (8) et la forme de la surface de butée (5) sont ajustées mutuellement à la forme de la surface (22) du col de prothèse (17), laquelle vient respectivement en interaction avec celles-ci.

3. Instrument d'extraction selon l'une ou l'autre des revendications précédentes, caractérisé en ce que la languette (8) est montée en déplacement dans un plan qui correspond au plan de courbure principale de la prothèse (15), et en ce qu'elle agit sur cette prothèse en particulier depuis l'extérieur par rapport à la courbure principale de celle-ci.

4. Instrument d'extraction selon l'une quelconque des revendications précédentes, caractérisé en ce que la languette agit sur le col de prothèse (17) avec une composante de force dirigée radialement vers l'extérieur, par rapport à l'axe médian géométrique de la tige (13) de l'instrument.

5. Instrument d'extraction selon l'une quelconque des revendications précédentes, caractérisé en ce que l'élément basculant (6) portant la languette (8) présente en tant qu'organe d'actionnement un élément de poignée (24) qui est fixé au-dessus du point de rotation (4) sur l'élément (6), et qui s'étend sensiblement parallèlement à l'axe longitudinal géométrique de la tige (13) de l'instrument (1), lorsque l'instrument d'extraction (1) a été fixé sur le col de prothèse (17).

6. Instrument d'extraction selon la revendication 5, caractérisé en ce qu'il est prévu un dispositif de verrouillage à l'extrémité libre (25) de l'élément de poignée (24).

7. Instrument d'extraction selon la revendication 6, caractérisé en ce que le dispositif de verrouillage présente un élément rotatif (26) monté excentriquement, qui s'engage dans sa position de verrouillage derrière l'extrémité libre (25) de l'élément de poignée (24), et en ce qu'il est prévu au moins une bosse (28) pour limiter l'angle de rotation.

8. Instrument d'extraction selon la revendication 7, caractérisé en ce que l'élément rotatif (26) est fixé sur la tige d'instrument (13), et en ce que l'extrémité libre (25) de l'élément de poignée (24) présente un évidement (27).

9. Instrument d'extraction selon l'une ou l'autre des revendications 7 et 8, caractérisé en ce que l'élément rotatif (26) est réalisé sensiblement en forme d'un disque, et en ce que la taille de sa surface frontale augmente tangentiellement en dépendance de l'angle de rotation en direction de la position de verrouillage.

10. Instrument d'extraction selon l'une quelconque des revendications précédentes, caractérisé en ce que l'élément basculant (6) portant la languette (8) présente une butée (9) supplémentaire située au-dessus de la languette (8) et venant en interaction avec le col (17) de la prothèse ou de la râpe à os, cette butée étant réalisée en particulier sous la forme d'un épaulement.

11. Instrument d'extraction selon l'une ou l'autre des revendications 5 et 10, caractérisé en ce que l'élément basculant (6) est monté en rotation autour d'un axe qui est dirigé perpendiculairement à l'axe médian géométrique de la tige (13) de l'instrument, et en particulier décalé radialement par rapport à celui-ci.

12. Instrument d'extraction selon l'une quelconque des revendications précédentes, caractérisé en ce que l'élément basculant (6) présente une surface de butée (7) située dans la région de la tige d'instrument (13), sur laquelle s'appuie l'arête (11) d'un élément d'actionnement (10) mobile en direction de la tige (13), l'arête (11) étant déplacée lors du déplacement de l'élément d'actionnement (10) en direction vers la monture (2) et entraînant l'élément basculant (6) de telle sorte que la languette (8) est pressée contre le col de prothèse (17).

13. Instrument d'extraction selon la revendication 12, caractérisé en ce que l'élément d'actionnement agencé sur la tige (13) est réalisé sous forme d'un cylindre moleté (10) pourvu d'un taraudage (12), dont l'arête (11) est en interaction avec la surface de butée (7) de l'élément basculant (6) portant la languette (8), de telle sorte que lors du déplacement du cylindre moleté (10), elle est déplacée en direction de la monture (2) et entraîne l'élément basculant (6) de telle sorte que la languette (8) est pressée contre le col de prothèse (17).

14. Instrument d'extraction selon l'une quelconque des revendications précédentes, caractérisé en ce qu'il est prévu une surface de butée supplémentaire (14) grâce à laquelle l'instrument d'extraction peut également être utilisé comme instrument d'enfoncement.

15. Instrument d'extraction selon la revendication 14, caractérisé en ce que la surface de butée supplémentaire (14) est située en prolongement des axes médians géométriques de la tige de la prothèse et de l'instrument.

16. Instrument d'extraction selon l'une ou l'autre des revendications 14 et 15, caractérisé en ce que la surface de butée supplémentaire (14) est réalisée sous forme d'une nervure.

17. Instrument d'extraction selon l'une quelconque des revendications 14 à 16, caractérisé en ce que la surface de butée supplémentaire (14) s'engage dans une surface de butée (23) de la prothèse (15), qui est située sur la face extérieure de la courbure maximum de la tige dans la région de la surface frontale de la tige de prothèse.

18. Instrument d'extraction selon l'une quelconque des revendications précédentes, caractérisé par l'application à une râpe à os pourvue des moyens de raccordement correspondants à ceux d'une prothèse.
